(19) Europäisches Patentamt
European Patent Office
Office européen des brevets

(11) Veröffentlichungsnummer: **0 138 760**
**B1**

(12) **EUROPÄISCHE PATENTSCHRIFT**

(45) Veröffentlichungstag der Patentschrift: **10.12.86**

(51) Int. Cl.⁴: **C 07 C 87/50**, C 07 C 85/00

(21) Anmeldenummer: **84810436.0**

(22) Anmeldetag: **10.09.84**

(54) **Verfahren zur Herstellung von N-substituierten Orthophenylendiaminen.**

(30) Priorität: **14.09.83 CH 5007/83**

(43) Veröffentlichungstag der Anmeldung: **24.04.85 Patentblatt 85/17**

(45) Bekanntmachung des Hinweises auf die Patenterteilung: **10.12.86 Patentblatt 86/50**

(84) Benannte Vertragsstaaten: **CH DE FR GB IT LI NL**

(56) Entgegenhaltungen:
**DE - C - 69 250**
**GB - A - 781 790**
**US - A - 3 290 376**

**JOURNAL OF ORGANOMETALLIC CHEMISTRY, Band 31, 1971, Lausanne M.I. BRUCE et al. "Ortho-metalation reaction III. Complexes from reactions between dodecarbonyl-triruthenium and azobenzene or o-semidine" Seiten 275-281**
**PATENTS ABSTRACTS OF JAPAN, unexamined applications, Sektion C, Band 3, Nr. 79, 6. Juli 1979 THE PATENT OFFICE JAPANESE GOVERNMENT Seite 92 C 51**

(73) Patentinhaber: **CIBA-GEIGY AG, Klybeckstrasse 141, CH-4002 Basel (CH)**

(72) Erfinder: **Spencer, Alwyn, Dr., 64B Kensington Gardens Sq., London W2 (GB)**

**EP 0 138 760 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein einstufiges Verfahren zur Herstellung von N-substituierten Orthophenylendiaminen aus Azobenzolen und einem sekundären Alkohol in Gegenwart von Rutheniumkatalysatoren und einer basischen Verbindung.

Aus J. Organometallic Chemistry, *31* (1971), 275 ist es bekannt, dass Azobenzol mit $Ru_3(CO)_{12}$ unter anderem zu Komplexen mit N-Phenylorthophenylendiamin führt. Das freie Diamin kann daraus durch Umsetzung mit $LiAlH_4$ erhalten werden.

Aufgabe vorliegender Erfindung ist es, ein einstufiges, katalytisches Verfahren zur Herstellung von N-substituierten Orthophenylendiaminen aufzuzeigen, bei dem billige und einfach zugängliche Ausgangsstoffe eingesetzt werden können.

Gegenstand vorliegender Erfindung ist ein Verfahren zur Herstellung von Orthophenylendiaminen der Formel I

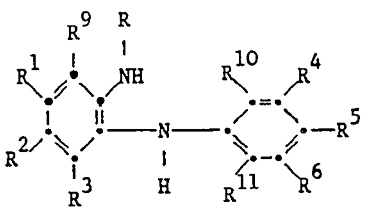

(I),

worin R für ein Wasserstoffatom oder eine Gruppe $R^7R^8CH-$ steht, in der $R^7$ und $R^8$ unabhängig voneinander unsubstituiertes oder substituiertes Alkyl, Cycloalkyl oder Alkoxyalkyl oder $R^7$ und $R^8$ zusammen $-(CH_2)_n$ mit n = 2 bis 12 sind, und $R^1$, $R^2$, $R^3$, $R^4$, $R^5$, $R^6$, $R^9$, $R^{10}$ und $R^{11}$ unabhängig voneinander für ein Wasserstoffatom, Halogen, Carboxylat, Alkyl, Alkoxy, Alkylthio, Alkoxyalkyl, Cycloalkyl, Aryl, Aralkyl oder je zwei benachbarte Gruppen von $R^1$, $R^2$, $R^3$, $R^9$ oder $R^4$, $R^5$, $R^6$, $R^{10}$, $R^{11}$, für $-CH=CH-CH=CH-$ stehen, oder Gemischen solcher Verbindungen der Formel I, in denen R ein Wasserstoffatom und die Gruppe $R^7R^8CH-$ bedeutet, dadurch gekennzeichnet, dass man ein Azobenzol der Formel

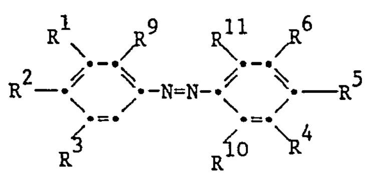

(II),

worin $R^1$ bis $R^6$ und $R^9$-$R^{11}$ die zuvor angegebene Bedeutung haben, in Gegenwart eines Rutheniumkatalysators und einer basischen Verbindung bei Temperaturen von mindestens 120° C mit einem sekundären Alkohol der Formel $R^7R^8CHOH$ umsetzt.

Bei $R^7$ und $R^8$ kann es sich um lineares oder verzweigtes Alkyl oder Alkoxyalkyl mit bevorzugt 1 bis 20, besonders 1 bis 12 C-Atomen und um Cycloalkyl mit bevorzugt 3 bis 10, besonders 3 bis 6 Ringkohlenstoffatomen handeln. Beispiele sind: Methyl, Äthyl, Propyl, Isopropyl, n-Butyl, Isobutyl, Tertiärbutyl, Pentyl, Hexyl, Heptyl, Octyl, Nonyl, Decyl, Dodecyl, Hexadecyl, Octadecyl, 2-Methyloxyäthyl, Methyloxymethyl, Äthyloxymethyl, Hexyloxymethyl, Cyclopropyl, Cyclobutyl, Cyclopentyl, Cyclohexyl, Cycloheptyl, Cyclooctyl. $R^7$ und $R^8$ zusammen können z.B. Dimethylen, Trimethylen, Tetramethylen oder Pentamethylen sein. In der $-(CH_2)_n$-Gruppe ist n bevorzugt 2 bis 8.

Geeignete Substituente für die Reste $R^7$ und $R^8$ sind zum Beispiel Alkyl mit bevorzugt 1 bis 6 C-Atomen wie Methyl, Äthyl, Propyl, Isopropyl, n-Butyl, Isobutyl, Tertiärbutyl, Pentyl, Hexyl; Alkoxy mit bevorzugt 1 bis 6 C-Atomen wie Methoxy, Äthoxy und Propoxy; Cycloalkyl mit bevorzugt 3 bis 7 Ring-C-Atomen, wie Cyclopropyl, Cyclopentyl und Cyclohexyl.

Von den Resten $R^1$ bis $R^6$ und $R^9$ bis $R^{11}$ bedeuten bevorzugt je einer von $R^1$ bis $R^3$ und $R^9$ und je einer von $R^4$ bis $R^6$ und $R^{10}$ und $R^{11}$ ein Wasserstoffatom. $R^1$ bis $R^6$ und $R^9$ bis $R^{11}$ als Halogen sind bevorzugt Fluor, Chlor oder Brom. $R^1$ bis $R^6$ und $R^9$ bis $R^{11}$ als Alkyl enthalten bevorzugt 1 bis 6, besonders 1 bis 4 C-Atome und als Cycloalkyl bevorzugt 3 bis 6 Ring-C-Atome; als Aryl bevorzugt 6 bis 12 C-Atome und sind als Aryl besonders Phenyl; als Aralkyl bevorzugt 7 bis 16 C-Atome und sind als Aralkyl besonders Phenylmethyl oder β-Phenyläthyl; als Alkoxy, Alkylthio und Alkoxyalkyl bevorzugt 1 bis 6 bzw. 2 bis 6 C-Atome und sind z.B. Methoxy, Äthoxy, Methoxymethyl, Methoxyäthyl; und bedeuten als Carboxylat z.B. einen Rest der Formel $-COOR^a$, worin $R^a$ Alkyl mit bevorzugt 1 bis 6 C-Atomen, Cyclohexyl oder Phenyl ist. $R^9$ bis $R^{11}$ sind bevorzugt ein Wasserstoffatom.

Beispiele für sekundäre Alkohole sind: Isopropanol, 2-Hydroxybutan, 2- oder 3-Hydroxypentan, 2- oder 3-Hydroxyhexan, 2-, 3- oder 4-Hydroxyheptan, 2-Hydroxyoctan, Cyclopropanol, Cyclobutanol, Cyclypentanol, Cyclohexanol, (α-Hydroxyäthyl)benzol, Diphenylhydroxymethan, 1,3-Dimethoxy-2-hydroxypropan. Die sekundären Alkohole sind bekannt, oder nach bekannten Verfahren herstellbar.

Beispiele für geeignete Azobenzole sind: Azobenzol, 4-Methylazobenzol, 4,4'-Dimethylazobenzol, 4,3'-Dimethylazobenzol, 4-Fluorazobenzol, 4-Fluor-4'-chlorazobenzol, 3-Bromazobenzol, 4,4'- oder 3,4'-Dichlorazobenzol, 3,5-Dichlorazobenzol, 3,5-Dimethylazobenzol, 3,3'-, 4,4'- und 3,5-Diäthylazobenzol, 4-Methoxyazobenzol, 4-(Methoxymethyl)azobenzol, 4-Phenylazobenzol, 3-(Methoxycarbonyl)azobenzol, 3-Benzylazobenzol, Naphthalinazobenzol, 4,4'-Difluorazobenzol, 3,3', 5,5'-Tetramethylazobenzol, 4-Chlor-4'-Methylazobenzol, 4-Fluor-4'-methylazobenzol, 4-Methyl-4'-(äthoxycarbonyl)azobenzol, 3,5-Dichlor-4'-methylazobenzol, 3,3'-Dimethylazobenzol, 4-Methyl-4-methoxyazobenzol. Bei den Azobenzolen handelt es sich ebenfalls um bekannte oder leicht herstellbare Verbindungen.

Katalysatoren werden bevorzugt in Mengen von 0,001 bis 20, besonders 0,01 bis 10 und insbesondere 0,01 bis 5 Mol-% verwendet. Es kann sich um heterogene Katalysatoren wie z.B. Ruthenium auf

einem geeigneten Trägermaterial wie Kohle oder bevorzugt um homogene Katalysatoren, die im Reaktionsgemisch löslich sind, handeln. Homogene Katalysatoren sind z.B. Verbindungen des Rutheniums, besonders dessen Salze von anorganischen oder organischen Säuren und dessen Komplexverbindungen. Die Komplexe können ein- oder mehrkernig sein und sie können polyfunktionelle, bevorzugt monofunktionelle Liganden enthalten. Solche Liganden sind z.B. in Advanced Inorganic Chemistry, 4th Edition, Verlag Wiley New York (1980), Seiten 107 bis 194 beschrieben. Die Verbindungen bzw. Komplexe können in allen Oxidationsstufen des Rutheniums vorliegen; bevorzugt sind die Oxidationsstufen 0, 1, 2, 3 und 4.

Geeignete Salze des Rutheniums leiten sich zum Beispiel von folgenden Säuren ab: Ameisensäure, Essigsäure, Benzoesäure, Toluolsulfonsäure, Phosphorsäure, Schwefelsäure, Perchlorsäure, Fluor-, Brom-, Jod- und besonders Chlorwasserstoffsäure. Die Salze können auch in ihrer hydratisierten Form eingesetzt werden. Besonders bevorzugt ist Rutheniumtrichloridtrihydrat. Unter den Komplexen sind solche mit Carbonylliganden besonders bevorzugt. Ein Beispiel ist Dodecacarbonyltriruthenium. In einer bevorzugten Ausführungsform des erfindungsgemässen Verfahrens werden die Carbonylkomplexe vor oder während der Reaktion aus z.B. den zuvor erwähnten Salzen gebildet, indem man die Reaktion unter reiner Kohlenmonoxidatmosphäre durchführt oder unter Gemischen von Kohlenmonoxid mit einem inerten Schutzgas wie z.B. Stickstoff oder einem Edelgas wie Helium oder Argon.

Als weitere Liganden kommen Verbindungen mit Donoratomen wie z.B. P, N, As, Sb, Bi, O, S, Se sowie Anionen organischer oder anorganischer Säuren in Frage. Beispiele sind die Arsine, Stibine, Bismuthine und besonders die Phosphine. Weitere Beispiele für Liganden sind Fluorid, Bromid, Chlorid, Jodid, Hydrid, das Trichlorzinn(II)-anion, Dimethylsulfoxid, Nitrosyl, das Acetat- und das Acetonylacetatanion. Als Liganden mit dreiwertigen Elementen der fünften Hauptgruppe des Periodensystems kommen besonders die Amine, Phosphine, Arsine, Stibine und Bismuthine in Frage, insbesondere solche mit Arylresten, wie z.B. Phenyl, das durch Halogen (F, Cl), $C_1$-$C_4$-Alkyl oder $C_1$-$C_4$-Alkoxy substituiert sein kann. Weitere Reste sind Alkyl mit bevorzugt 1 bis 12 C-Atomen, Cycloalkyl, z.B. Cyclohexyl, und Aralkyl, z.B. Benzyl. Bevorzugte Liganden aus dieser Gruppe sind die Phosphine. Beispiele sind:

Triphenylphosphin, Tri(o-toluyl)phosphin, Tri(p-toluyl)phosphin, Tri(p-fluorphenyl)phosphin, Tri(p-methoxyphenyl)phosphin, Tertiärbutyldiphenylphosphin, Tricyclohexylphosphin, Tetraphenyldiphosphin, Triphenylamin, Triphenylstibin. Ferner sind auch Phosphite geeignet, besonders solche mit Arylresten wie z.B. Phenyl. Ein Beispiel ist Triphenylphosphit. Die Komplexe können gleiche oder verschiedene Liganden enthalten. Bevorzugt enthalten die Komplexe Phosphin- und CO-Liganden.

Die Komplexe können zu Beginn der Reaktion als isolierte Verbindungen zugegeben werden. Es hat sich als zweckmässig erwiesen, die Komplexe vor Reaktionsbeginn aus Rutheniumverbindungen (z.B. deren Salzen) durch Zugabe der ligandenbildenden Verbindungen, gegebenenfalls in CO-Atmosphäre, herzustellen und das erfindungsgemässe Verfahren unter Zugabe der Reaktanden weiterzuführen.

Die ligandenbildenden Verbindungen können in äquimolaren Mengen oder einem Überschuss zugegeben werden. Das molare Verhältnis von Rutheniumverbindungen zu Liganden beträgt bevorzugt 10 zu 1, besonders 6 bis 2.

In einer bevorzugten Ausführungsform des Verfahrens werden Rutheniumverbindungen eingesetzt, besonders deren Halogenide, und vorteilhaft wird die Reaktion zusätzlich unter CO-Atmosphäre durchgeführt.

Geeignete basische Verbindungen sind z.B. primäre, sekundäre und besonders tertiäre Amine. Beispiele für Amine sind Methylamin, Dimethylamin, Trimethylamin, Cyclohexylamin, Cyclohexyldimethylamin, Morpholin, N-Methylmorpholin, Piperidin, N-Methylpiperidin, Pyrrolidin, N-Methylpyrrolidin, Benzyldimethylamin.

Bevorzugte basische Verbindungen sind Metallcarbonsäuresalze besonders die Erdalkalimetallsalze, z.B. die Calcium und Strontiumsalze, und insbesondere die Alkalimetallsalze von Carbonsäuren. Bevorzugte Alkalimetalle sind Kalium und besonders Natrium und Lithium. Beispiele sind:

Natriumhydrogencarbonat, Lithiumacetat, Lithiumpropionat, Lithiumformiat, Lithiumbenzoat, Natriumacetat und Natriumbenzoat.

Die basische Verbindung wird bevorzugt in einer Menge von mindestens 0,1 Mol-%, bezogen auf das Azobenzol der Formel II, eingesetzt und es können bis zu stöchiometrische Mengen oder mehr verwendet werden.

Die Reaktanden werden bevorzugt in einem Molverhältnis von sekundärem Alkohol zu Azobenzol von mindestens 1:1 eingesetzt. Das Verhältnis kann bis 8:1 und mehr betragen, wobei ein Überschuss gleichzeitig als Lösungsmittel dienen kann.

In einer bevorzugten Ausführungsform beträgt das Molverhältnis von sekundärem Alkohol zu Azobenzol 1,0:1 bis 2,5:1 und die basische Verbindung wird in bis zu stöchiometrischen Mengen zugegeben. Hierbei erhält man Verbindungen der Formel I, worin R ein Wasserstoffatom bedeutet, oft im Gemisch mit solchen Verbindungen der Formel I, worin R eine $R^7R^8CH$-Gruppe ist. Die Isolierung von Einzelverbindungen aus den Gemischen kann dann nach üblichen Methoden erfolgen. In einer weiteren bevorzugten Ausführungsform des Verfahrens beträgt das Molverhältnis von sekundärem Alkohol zu Azobenzol 3:1 bis 8:1, besonders 4:1 bis 6:1, und die Menge der basischen Verbindung beträgt 0,1 bis 20 Mol-%, besonders 1 bis 10 Mol-%. Hierbei werden reine Verbindungen der Formel I gebildet, bei denen R die $R^7R^8CH$-Gruppe ist, oder Gemische, die überwiegend solche Verbindungen im Gemisch mit Verbindungen enthalten, worin R ein Wasserstoffatom bedeutet.

Vorteilhaft wird ein inertes Lösungsmittel mitverwendet. Besonders geeignet sind polare aprotische Lösungsmittel. Bevorzugt sind N-alkylierte Säureamide. Ferner sind infolge der Reaktionstemperaturen höhersiedende Lösungsmittel bevorzugt, um das Verfahren bei Normaldruck durchführen zu können.

Beispiele für Lösungsmittel sind: Kohlenwasserstoffe wie Benzol, Toluol, Chlorbenzol, Dichlorbenzol, Benzonitril und Tetralin, Sulfone wie Tetramethylensulfon, Sulfoxide wie Dimethylsulfoxid, tertiäre Amine wie Diphenylamin, Cyclohexyldimethylamin und N-Methylpiperidin, Äther wie Dioxan, Diäthylenglykoldimethyl- oder -diäthyläther und Triäthylenglykoldimethyläther, und lineare oder cyclische N-alkylierte Säureamide wie Tetramethylharnstoff, Dimethylformamid, Dimethylacetamid, Diäthylacetamid, N-Methylpyrrolidon, N-Formylmorpholin, N-Formylpiperidin, Hexamethylphosphorsäuretriamid, Ester von Phosphorsäure oder Kohlensäure wie z.B. Triäthylphosphat und Propylencarbonat. Bevorzugte Lösungsmittel sind Diäthylformamid, Dimethylacetamid, N-Formylmorpholin und besonders Tetramethylharnstoff und auch 1,3-Dimethyl-2-imidazolidon.

Das Verfahren wird in hierfür üblichen Einrichtungen durchgeführt, indem man die Reaktionspartner, den Katalysator, die basische Verbindung und gegebenenfalls ein Lösungsmittel zusammenmischt und danach das Reaktionsgemisch auf die gewünschte Reaktionstemperatur erwärmt. Diese beträgt bevorzugt 120 bis 250° C, besonders 150 bis 230° C, und insbesondere 150 bis 220° C. Das Verfahren kann unter Normaldruck oder Überdruck durchgeführt werden.

Die Isolierung der Reaktionsprodukte erfolgt in üblicher Weise zum Beispiel durch Kristallisation oder Destillation, gegebenenfalls nach Entfernen des Lösungsmittels.

Mit dem erfindungsgemässen Verfahren erhält man auf einfache Weise in guten Ausbeuten und guter Reinheit aus billigen Ausgangsstoffen N-phenylierte Orthophenylendiamine (Verbindung A), oder solche Orthophenylendiamine, die zusätzlich am zweiten N-Atom durch eine $R^7R^8CH$-Gruppe substituiert sind (Verbindung B). Verbindungen A sind wertvolle Zwischenprodukte zur Herstellung von N-Phenylbenzimidazolen, die als Blattfungizide wirksam sind (vgl. CH-PS 478256). Verbindungen B können z.B. als Antioxidantien für Benzin verwendet werden (vgl. US-PS 3290376).

Die nachfolgenden Beispiele erläutern die Erfindung näher. Die Ausbeuten sind auf das eingesetzte Azobenzol bezogen. Zur Kennzeichnung der Stellung der Substituenten gilt folgende Numerierung in den Diaminen der Formel I

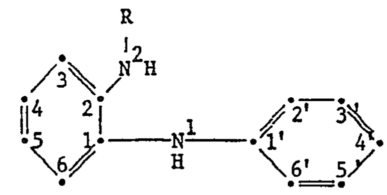

*Beispiel 1:*

In einem Druckrohr wird 25 ml Tetramethylharnstoff vorgelegt und Kohlenmonoxid wird unter Rühren durchgeleitet. Es werden 9,1 g (50 mMol) Azobenzol, 7,67 ml (100 mMol) Isopropanol, 3,3 g (32 mMol) Lithiumacetatdihydrat, 0,1308 g Rutheniumtrichloridtrihydrat und 0,524 g (2 mMol) Triphenylphosphin zugegeben. Das Rohr wird unter Kohlenmonoxid bei Normaldruck verschlossen und dann während 8 Std bei 180° C gerührt. Nach dem Entfernen des Lösungsmittels wird das Produkt am Hochvakuum destilliert und anschliessend aus einem Gemisch aus 50 ml Cyclohexan und 50 ml Hexan umkristallisiert. Man erhält 3,9 g (21,1 mMol) N-Phenyl-1,2-Benzoldiamin als weisse Kristalle vom F.p. 79,6° C, entsprechend einer Ausbeute von 42% der Theorie.

*Beispiel 2:*

Es wird wie im Beispiel 1 beschrieben vorgegangen jedoch unter Verwendung von 11,51 ml (150 mMol) Isopropanol und 0,33 g (3,2 mMol) Lithiumacetat Dihydrat. Nach der Destillation wird das Rohprodukt auf Kieselgel in Methylenchlorid chromatographiert und nochmals am Hochvakuum destilliert. Man erhält 4,0 g (17,7 mMol) $N^2$-Isopropyl-$N^1$-Phenyl-1,2-benzoldiamin als gelbe Flüssigkeit vom K.p. 118-22° C/0,1 mm Hg, entsprechend einer Ausbeute von 35% der Theorie.

*Beispiele 3-17:*

Es wird wie im Beispiel 1 beschrieben vorgegangen, jedoch unter Verwendung von 12,5 ml Tetramethylharnstoff, 4,55 g (25 mMol) Azobenzol, 0,0654 g (0,25 mMol) Rutheniumtrichloridtrihydrat, 0,262 g (1 mMol) Triphenylphosphin und den in der Tabelle angegebenen Mengen von Isopropanol und Natriumacetat (wasserfrei). Nach einer Reaktionszeit von 8 Std. bei 180° werden die Ausbeuten der beiden Produkte N-Phenyl-1,2-benzoldiamin (A) und $N^2$-Isopropyl-$N^1$-phenyl-1,2-benzoldiamin (B) gaschromatographisch bestimmt.

| Beispiel-Nr. | Isopropanol (mMol) | Natriumacetat (mMol) | Ausbeute A | (%) B |
|---|---|---|---|---|
| 3 | 25 | 1 | 19 | 5 |
| 4 | 25 | 10 | 17 | 5 |
| 5 | 25 | 25 | 21 | 6 |

| Beispiel-Nr. | Isopropanol (mMol) | Natriumacetat (mMol) | Ausbeute A | (%) B |
|---|---|---|---|---|
| 6 | 35 | 1 | 35 | 15 |
| 7 | 40 | 10 | 31 | 17 |
| 8 | 40 | 25 | 24 | 8 |
| 9 | 40 | 30 | 28 | 19 |
| 10 | 50 | 15 | 28 | 17 |
| 11 | 50 | 25 | 33 | 25 |
| 12 | 50 | 30 | 32 | 22 |
| 13 | 75 | 1 | 14 | 61 |
| 14 | 75 | 10 | 32 | 31 |
| 15 | 125 | 0 | 30 | 27 |
| 16 | 125 | 1 | 0 | 79 |
| 17 | 125 | 25 | 27 | 30 |

*Beispiel 18:*

Es wird wie im Beispiel 1 beschrieben vorgegangen, jedoch unter Verwendung von 12,55 g (50 mMol) 4,4'-Dichlorazobenzol anstelle vom Azobenzol. Nach der Destillation wird das Rohprodukt aus einem Gemisch aus 20 ml Cyclohexan und 5 ml Tetrachlorkohlenstoff umkristallisiert. Man erhält 2,55 g (10,1 mMol) $N^1$-(4-Chlorphenyl)-5-chlor-1,2-benzoldiamin als weisse Kristalle vom F.p. 92,3° C, entsprechend einer Ausbeute von 20% der Theorie.

*Beispiel 19:*

Es wird wie im Beispiel 1 beschrieben vorgegangen, jedoch unter Verwendung von 10,9 g (50 mMol) 4,4'-Difluorazobenzol anstelle vom Azobenzol. Nach der Destillation wird das Rohprodukt in Dichlormethan auf Kieselgel chromatographiert und nochmals am Hochvakuum destilliert. Man erhält 4,2 g (19,1 mMol) $N^1$-(4-Fluorphenyl)-5-Fluor-1,2-benzoldiamin als gelbe Flüssigkeit vom K.p. 129-31° C/0,1 mm Hg, entsprechend einer Ausbeute von 38% der Theorie.

*Beispiel 20:*

Es wird wie in Beispiel 1 beschrieben vorgegangen, jedoch unter Verwendung von 10,5 g (50 mMol) 4,4'-Dimethylazobenzol anstelle vom Azobenzol. Nach der Destillation wird das Rohprodukt aus 150 ml n-Hexan umkristallisiert. Man erhält 3,7 g (17,5 mMol) $N^1$-(4-Methylphenyl)-5-methyl-1,2-benzoldiamin als weisse Kristalle vom F.p. 108,1° C, entsprechend einer Ausbeute von 35% der Theorie.

*Beispiel 21:*

Es wird wie im Beispiel 1 beschrieben vorgegangen, jedoch unter Verwendung von 12,55 g (50 mMol) 4,4'-Dichlorazobenzol anstelle von Azobenzol, und mit 19,2 ml (250 mMol) Isopropanol und 0,132 g (2 mMol) Lithiumacetat (wasserfrei). Nach der Destillation und dem Chromatographieren auf Kieselgel in Toluol wird das Produkt aus 30 ml n-Pentan umkristallisiert. Man erhält 6,6 g (22,4 mMol) $N^2$-Isopropyl-$N^1$-(4-chlorphenyl)-5-chlor-1,2-benzoldiamin als weisse Kristalle vom F.p. 70,7° C, entsprechend einer Ausbeute von 45% der Theorie.

*Beispiel 22:*

Es wird wie im Beispiel 21 beschrieben vorgegangen, jedoch unter Verwendung von 10,9 g (50 mMol) 4,4'-Difluorazobenzol anstelle von Azobenzol. Nach der Destillation und Chromatographie auf Kieselgel in Dichlormethan wird das Rohprodukt aus 50 ml n-Pentan umkristallisiert. Man erhält 7,3 g (27,9 mMol) $N^2$-Isopropyl-$N^1$-(4-fluorphenyl)-5-fluor-1,2-benzoldiamin als weisse Kristalle vom F.p. 81,3° C entsprechend einer Ausbeute von 56% der Theorie.

*Beispiele 23:*

Es wird wie im Beispiel 21 beschrieben vorgegangen, jedoch unter Verwendung von 10,5 g (50 mMol) 4,4'-Dimethylazobenzol anstelle von Azobenzol. Nach Destillation und Chromatographieren auf Kieselgel in Toluol wird das Produkt nochmals destilliert. Man erhält 6,9 g (27,2 mMol) $N^2$-Isopropyl-N'-(4-methylphenyl)-5-methyl-1,2-benzoldiamin als gelbe Flüssigkeit von K.p. 128-32° C/0,1 mm Hg entsprechend einer Ausbeute von 54% der Theorie.

*Beispiele 24-26:*

Es wird wie in Beispiel 1 beschrieben vorgegangen, jedoch unter Verwendung von 12,5 ml Tetramethylharnstoff, 25 mMol der in der Tabelle angegebenen Azobenzole, 3,84 ml (50 mMol) Isopropanol, 1,65 g (25 mMol) Lithiumacetat, 0,065 g (0,25 mMol) Rutheniumtrichloridtrihydrat und 0,262 g (1 mMol) Triphenylphosphin. Die Ergebnisse sind in der Tabelle dargestellt.

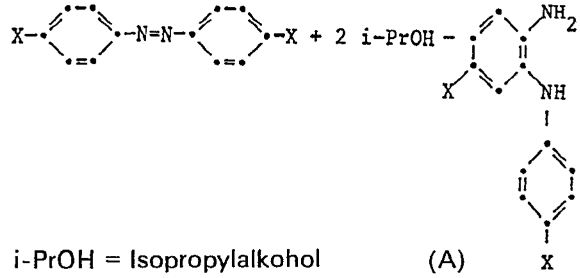


i-PrOH = Isopropylalkohol (A)

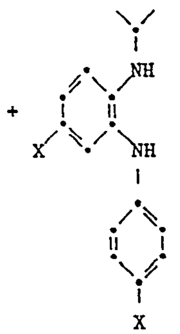

(B)

| Beispiel Nr. | X | Ausbeute (%) | |
|---|---|---|---|
| | | A | B |
| 24 | Cl | 30 | 14 |
| 25 | F | 32 | 15 |
| 26 | Me | 35 | 24 |

*Beispiele 27, 28:*

Es wird wie in Beispiel 24 beschrieben vorgegangen, jedoch unter Verwendung von 4,55 g (25 mMol) Azobenzol, 1,92 ml (25 mMol) Isopropanol und 1 mMol der in der Tabelle angegebenen Phosphorliganden. Die Ausbeuten werden gaschromatographisch bestimmt. N-Phenyl-1,2-benzoldiamin (A), $N^1$-Phenyl-$N^2$-isopropyl-1,2-benzoldiamin (B).

| Beispiele Nr. | Phosphor-liganden | Ausbeute (%) | |
|---|---|---|---|
| | | A | B |
| 27 | Tri(p-tolyl)-phosphin | 20 | 0 |
| 28 | Tri(p-fluor-phenyl)-phosphin | 7 | 0 |

*Beispiele 29, 30:*

Es wird wie in Beispiel 27 beschrieben vorgegangen, jedoch unter Verwendung von 9,6 ml (125 mMol) Isopropanol, 0,066 g (1 mMol) Lithiumacetat und 1 mMol der in der Tabelle angegebenen Phosphorliganden. Die Ausbeuten werden gaschromatographisch bestimmt.

| Beispiele Nr. | Phosphor-liganden | Ausbeute (%) | |
|---|---|---|---|
| | | A | B |
| 29 | Tri(p-tolyl)-phosphin | 0 | 62 |
| 30 | Tri(p-fluor-phenyl)-phosphin | 35 | 36 |

*Beispiele 31-37:*

Es wird wie in Beispiel 1 beschrieben vorgegangen, jedoch unter Verwendung von 4,55 g (25 mMol) Azobenzol, 3,84 ml (50 mMol) Isopropanol, 0,0654 g (0,25 mMol) Rutheniumtrichloridtrihydrat, 0,262 g (1 mMol) Triphenylphosphin und gemäss der Tabelle 12,5 ml Lösungsmittel, 25 mMol Base, bei einer Reaktionszeit von 8 Std. bei der in der Tabelle angegebenen Temperaturen.

*Beispiel 38:*

Es wird wie im Beispiel 1 beschrieben vorgegangen jedoch unter Verwendung von 10,58 ml (100 mMol) cyclohexanol anstelle vom Isopropanol. Nach der Destillation und dem Chromatographieren auf Kieselgel in Dichlormethan wird das Produkt nochmals am Hochvakuum destilliert. Man erhält 3,38 g (18,4 mMol) N-Phenyl-1,2-benzoldiamin als gelbe Flüssigkeit von K.p. 139-41° C/0,1 mm Hg, entsprechend einer Ausbeute von 37% der Theorie.

*Beispiel 39:*

Es wird wie im Beispiel 21 beschrieben vorgegangen jedoch unter Verwendung von 9,1 g (50 mMol) Azobenzol und 22,97 ml (250 mMol) 2-Butanol anstelle von Dichlorazobenzol und Isopropanol. Nach der Destillation wird das Rohprodukt auf Kieselgel in Toluol aufgetrennt und die beiden Rohprodukte nochmals destilliert. Man erhält zusätzlich zum N-Phenyl-1,2-benzoldiamin (Ausbeute 30%), 1,49 g (6,2 mMol) $N^2$-Phenyl-$N^1$-(2-butyl)-1,2-benzoldiamin als gelbe Flüssigkeit von K.p. 139-142° C/0,1 mm Hg, entsprechend einer Ausbeute von 6% der Theorie.

*Beispiel 40:*

Es wird wie im Beispiel 39 beschrieben vorgegangen, jedoch unter Verwendung von 31,3 ml (250 mMol) 3-Hexanol anstelle von 2-Butanol. Als Produkt erhält man N-Phenyl-1,2-benzoldiamin in einer Ausbeute von 30% der Theorie.

*Beispiel 41:*

Es wird wie im Beispiel 39 beschrieben vorgangen jedoch unter Verwendung von 9,8 g (50 mMol) 4-Methylazobenzol und 19,15 ml (250 mMol) Isopropanol anstelle vom Azobenzol und 2-Butanol. Nach der Destillation und dem Chromatographieren auf Kieselgel in Chloroform wird das Produkt nochmals destilliert. Man erhält 6,3 g (26,25 mMol) eines Isomerengemisches bestehend zu 35% aus $N^2$-Phenyl-$N^1$-Isopropyl-5-methyl-1,2-benzoldiamin und zu 65% aus $N^2$-(4-Methylphenyl)-$N^1$-isopropyl-1,2-benzoldiamin von K.p. 122-28° C/0,1 mm Hg entsprechend einer Gesamtausbeute von 53% der Theorie.

*Beispiel 42:*

Es wird wie in Beispiel 16 beschrieben vorgegangen jedoch unter Verwendung von 5,25 g (25 mMol) 2,2'-Dimethylazobenzol anstelle von Azobenzol. Nach 8 Std. bei 180° C und Aufarbeitung gemäss Beispiel 19 erhält man 0,44 g

(1,73 mMol) $N^1$-(2'-Methylphenyl)-$N^2$-Isopropyl-4-methyl-1,2-benzoldiamin als rotes Öl von K.p. 120° C/0,1 mm Hg.

## Patentansprüche

1. Verfahren zur Herstellung von Orthophenylendiaminen der Formel I

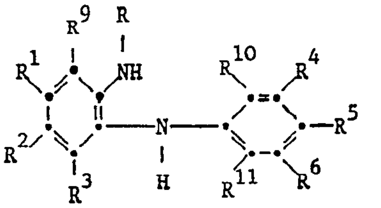

(I),

worin R für ein Wasserstoffatom oder eine Gruppe $R^7R^8CH-$ steht, in der $R^7$ und $R^8$ unabhängig voneinander unsubstituiertes oder substituiertes Alkyl, Alkoxyalkyl oder Cycloalkyl oder $R^7$ und $R^8$ zusammen $-(CH_2)_n$ mit n = 2 bis 12 sind, und $R^1$, $R^2$, $R^3$, $R^4$, $R^5$, $R^6$, $R^9$, $R^{10}$ und $R^{11}$ unabhängig voneinander für ein Wasserstoffatom, Halogen, Carboxylat, Alkyl, Alkoxy, Alkylthio, Alkoxyalkyl, Cycloalkyl, Aryl, Aralkyl oder je zwei benachbarte Gruppen von $R^1$, $R^2$, $R^3$, $R^9$ oder $R^4$, $R^5$, $R^6$, $R^{10}$, $R^{11}$ für $-CH=CH-CH=CH-$ stehen, oder Gemischen solcher Verbindungen der Formel I, in denen R ein Wasserstoffatom und die Gruppe $R^7R^8CH-$ bedeutet, dadurch gekennzeichnet, dass man ein Azobenzol der Formel

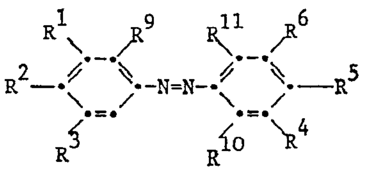

(II),

worin $R^1$ bis $R^6$ und $R^9$ bis $R^{11}$ die zuvor angegebene Bedeutung haben, in Gegenwart eines Rutheniumkatalysators und einer basischen Verbindung bei Temperaturen von mindestens 120° C mit einem sekundären Alkohol der Formel $R^7R^8CHOH$ umsetzt.

2. Verfahren gemäss Anspruch 1, dadurch gekennzeichnet, dass $R^7$ und $R^8$ unsubstituiertes oder substituiertes Alkyl oder Alkoxyalkyl mit 1 bis 20 C-Atomen, Cycloalkyl mit 3 bis 10 Ringkohlenstoffatomen, oder $R^7$ und $R^8$ zusammen $-(CH_2)_n$ mit n = 2 bis 8 sind.

3. Verfahren gemäss Anspruch 1, dadurch gekennzeichnet, dass die Substituenten für $R^7$ und $R^8$ ausgewählt sind aus Alkyl, Cycloalkyl oder Alkoxy.

4. Verfahren gemäss Anspruch 1, dadurch gekennzeichnet, dass als Katalysator Salze oder Komplexverbindungen des Rutheniums verwendet werden.

5. Verfahren gemäss Anspruch 1, dadurch gekennzeichnet, dass der Rutheniumkatalysator in einer Menge von 0,001-20 Mol-%, bezogen auf das Azobenzol der Formel II, eingesetzt wird.

6. Verfahren gemäss Anspruch 1, dadurch gekennzeichnet, dass die Umsetzung in einem polaren aprotischen Lösungsmittel durchgeführt wird.

7. Verfahren gemäss Anspruch 6, dadurch gekennzeichnet, dass das Lösungsmittel ein N-alkyliertes Säureamid, insbesondere Tetramethylharnstoff ist.

8. Verfahren gemäss Anspruch 1, dadurch gekennzeichnet, dass es bei einer Temperatur von 120 bis 250° C durchgeführt wird.

9. Verfahren gemäss Anspruch 1, dadurch gekennzeichnet, dass das Molverhältnis von sekundärem Alkohol zu Azobenzol mindestens 1:1 beträgt.

10. Verfahren gemäss Anspruch 1, dadurch gekennzeichnet, dass es sich bei der basischen Verbindung um ein tertiäres Amin oder ein Alkali- oder Erdalkalisalz einer Carbonsäure handelt.

11. Verfahren gemäss Anspruch 10, dadurch gekennzeichnet, dass die basische Verbindung in einer Menge von mindestens 0,1 Mol-%, bezogen auf das Azobenzol der Formel II, eingesetzt wird.

12. Verfahren gemäss Anspruch 9, dadurch gekennzeichnet, dass das Molverhältnis von sekundärem Alkohol zu Azobenzol 1,0:1 bis 2,5:1 beträgt und die basische Verbindung in bis zu stöchiometrischen Mengen, bezogen auf das Azobenzol, eingesetzt wird.

13. Verfahren gemäss Anspruch 9, dadurch gekennzeichnet, dass das Molverhältnis von sekundärem Alkohol zu Azobenzol 3:1 bis 8:1 beträgt und die basische Verbindung in einer Menge von 0,1 bis 20 Mol-% eingesetzt wird.

## Claims

1. A process for the preparation of an orthophenylenediamine of the formula I

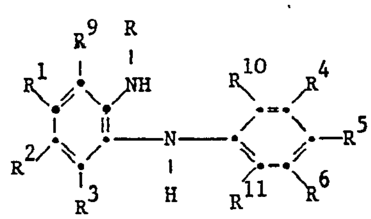

(I),

in which R is a hydrogen atom or a group $R^7R^8CH-$, in which each of $R^7$ and $R^8$ independently of the other is unsubstituted or substituted alkyl, alkoxyalkyl or cycloalkyl, or $R^7$ and $R^8$ together are $-(CH_2)_n$, where n = 2 to 12, and each of $R^1$, $R^2$, $R^3$, $R^4$, $R^5$, $R^6$, $R^9$, $R^{10}$ and $R^{11}$ independently is a hydrogen atom, halogen carboxylate, alkyl, alkoxy, alkylthio, alkoxyalkyl, cycloalkyl, aryl or aralkyl, or each pair of adjacent groups $R^1$, $R^2$, $R^3$ and $R^9$ or $R^4$, $R^5$, $R^6$, $R^{10}$ and $R^{11}$ is $-CH=CH-CH=CH-$, or mixtures of those compounds of the formula I in which R is a hydrogen atom or a group $R^7R^8CH-$, which process comprises reacting an azobenzene of the formula

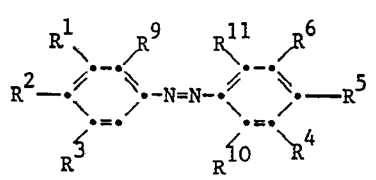

(II),

in which $R^1$ to $R^6$ and $R^9$ to $R^{11}$ are as defined above, with a secondary alcohol of the formula $R^7R^8CHOH$ in the presence of a ruthenium catalyst and a basic compound at temperatures of at least 120° C.

2. A process according to claim 1, wherein $R^7$ and $R^8$ are unsubstituted or substituted alkyl or alkoxyalkyl with 1 to 20 C atoms or cycloalkyl with 3 to 10 ring carbon atoms, or $R^7$ and $R^8$ together are $-(CH_2)_n$, where n = 2 to 8.

3. A process according to claim 1, wherein the substituents for $R^7$ and $R^8$ are chosen from alkyl, cycloalkyl and alkoxy.

4. A process according to claim 1, wherein the catalyst is a salt or a complex compound of ruthenium.

5. A process according to claim 1, wherein the ruthenium catalyst is used in an amount of 0.001 to 20 mol-%, based on the azobenzene of the formula II.

6. A process according to claim 1, wherein the reaction is carried out in a polar aprotic solvent.

7. A process according to claim 6, wherein the solvent is an N-alkylated acid amide, in particular tetramethylurea.

8. A process according to claim 1, carried out at a temperature in the range from 120 to 250° C.

9. A process according to claim 1, wherein the molar ratio of secondary alcohol to azobenzene is at least 1:1.

10. A process according to claim 1, wherein the basic compound is a tertiary amine or an alkali metal or alkaline earth metal salt of a carboxylic acid.

11. A process according to claim 10, wherein the basic compound is used in an amount of at least 0.1 mol-%, based on the azobenzene of the formula II.

12. A process according to claim 9, wherein the molar ratio of secondary alcohol to azobenzene is 1.0:1 to 2.5:1, and the basic compound is used in up to stoichiometric amounts, based on the azobenzene.

13. A process according to claim 9, wherein the molar ratio of secondary alcohol to azobenzene is 3:1 to 8:1 and the basic compound is used in an amount of 0.1 to 20 mol-%.

## Revendications

1. Procédé de préparation d'o-phénylène-diamines répondant à la formule (I):

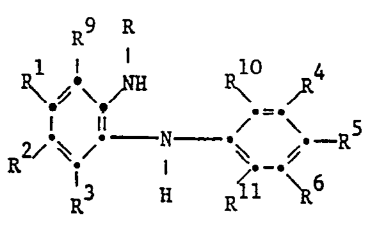

(I),

dans laquelle R représente un atome d'hydrogène ou un radical $R^7R^8CH-$ dont les symboles $R^7$ et $R^8$ représentent chacun, indépendamment l'un de l'autre, un radical alkyle, alcoxy-alkyle ou cycloalkyle substitué ou non, ou $R^7$ et $R^8$ forment ensemble un groupement $-(CH_2)_n$ dans lequel n désigne un nombre de 2 à 12, et $R^1$, $R^2$, $R^3$, $R^4$, $R^5$, $R^6$, $R^9$, $R^{10}$ et $R^{11}$ représentent chacun, indépendamment les uns des autres, un atome d'hydrogène ou d'halogène ou un radical carboxylate, alkyle, alcoxy, alkylthio, alcoxyalkyle, cycloalkyle, aryle ou aralkyle, ou encore deux radicaux voisins pris parmi $R^1$, $R^2$, $R^3$, $R^9$ ou deux radicaux voisins pris parmi $R^4$, $R^5$, $R^6$, $R^{10}$ et $R^{11}$ peuvent former un radical $-CH=CH-CH=CH-$, ou de mélanges de tels composés de formule (I) dans lesquels R représente un atome d'hydrogène et un radical $R^7R^8CH-$, procédé caractérisé en ce qu'on fait réagir un azobenzène répondant à la formule (II):

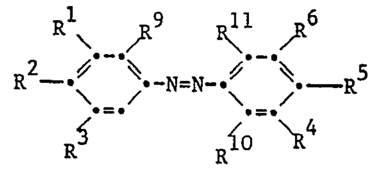

(II),

dans laquelle $R^1$ à $R^6$ et $R^9$ à $R^{11}$ ont les significations précédemment données, en présence d'un catalyseur au ruthénium et d'un composé basique, à des températures d'au moins 120° C, avec un alcool secondaire de formule $R^7R^8CHOH$.

2. Procédé selon la revendication 1 caractérisé en ce que $R^7$ et $R^8$ représentent chacun un radical alkyle ou alcoxyalkyle, substitué ou non, qui contient de 1 à 20 atomes de carbone, ou un radical cycloalkyle qui contient de 3 à 10 atomes de carbone dans le cycle, ou $R^7$ et $R^8$ forment ensemble un radical $-(CH_2)_n$ dans lequel n est un nombre de 2 à 8.

3. Procédé selon la revendication 1 caractérisé en ce que les substituants $R^7$ et $R^8$ sont choisis dans l'ensemble constitué par des radicaux alkyles, cycloalkyles et alcoxy.

4. Procédé selon la revendication 1 caractérisé en ce qu'on utilise, comme catalyseurs, des sels ou des complexes du ruthénium.

5. Procédé selon la revendication 1 caractérisé en ce que le catalyseur au ruthénium est mis en jeu en une quantité de 0,001 à 20% en moles par rapport à l'azobenzène de formule II.

6. Procédé selon la revendication 1 caractérisé en ce que la réaction est effectuée dans un solvant aprotique polaire.

7. Procédé selon la revendication 6 caractérisé en ce que le solvant est un amide alkylé à l'azote, plus spécialement le tétraméthylurée.

8. Procédé selon la revendication 1 caractérisé en ce qu'il est exécuté à une température de 120 à 250° C.

9. Procédé selon la revendication 1 caractérisé en ce que le rapport molaire de l'alcool secondaire à l'azobenzène est d'au moins 1:1.

10. Procédé selon la revendication 1 caractérisé en ce que le composé basique est une amine tertiaire ou un sel de métal alcalin ou de métal alcalino-terreux d'un acide carboxylique.

11. Procédé selon la revendication 10 caractérisé en ce que le composé basique est mis en jeu en une quantité d'au moins 0,1% en moles par rapport à l'azobenzène de formule (II).

12. Procédé selon la revendication 9 caractérisé en ce que le rapport molaire de l'alcool secondaire à l'azobenzène est compris entre 1,0:1 et 2,5:1 et en ce que le composé basique est mis en jeu en une quantité pouvant aller jusqu'à la quantité stœchiométrique, par rapport à l'azobenzène.

13. Procédé selon la revendication 9 caractérisé en ce que le rapport molaire de l'alcool secondaire à l'azobenzène est compris entre 3:1 et 8:1 et en ce que le composé basique est mis en jeu en une quantité de 0,1 à 20% en moles.